# EUROPEAN PATENT SPECIFICATION

(11) **EP 2 441 828 B1**
(45) Date of publication and mention of the grant of the patent: **18.02.2015**
(21) Application number: 10187730.6
(22) Date of filing: 15.10.2010
(51) Int. Cl.: C12N 1/12, C12N 15/63, C12P 5/00

(54) **Algal bio-flocculation by inactivation of photoreceptors**
Bioflockung von Algen mittels Inaktivierung der Fotorezeptoren
Biofloculation algale par inactivation de photorécepteurs

(43) Date of publication of application: 18.04.2012
(73) Proprietor: Stazione Zoologica "Anton Dohrn", 80121 Napoli (IT)
(72) Inventor: Falciatore, Angela, 80121 Napoli (IT); Bowler, Chris, 80121 Napoli (IT); Raniello, Raffaella, 80121 Napoli (IT)
(74) Representative: Capasso, Olga

(56) References cited:
- DE RISO VALENTINA ET AL: "Gene silencing in the marine diatom Phaeodactylum tricornutum", NUCLEIC ACIDS RESEARCH, vol. 37, no. 14, August 2009 (2009-08), XP002622830, ISSN: 0305-1048
- KNUCKEY R M ET AL: "Production of microalgal concentrates by flocculation and their assessment as aquaculture feeds", AQUACULTURAL ENGINEERING, ELSEVIER SCIENCE PUBLISHERS LTD, vol. 35, no. 3, 1 October 2006 (2006-10-01), pages 300-313, XP025087462, ISSN: 0144-8609, DOI: DOI:10.1016/J.AQUAENG.2006.04.001 [retrieved on 2006-10-01]

### SEQUENCE LISTING

<110> Stazione Zoologica Anton Dohrn
<120> Algal bio-flocculation by inactivation of photoreceptors
<130> BE 109560
<160> 17
<170> PatentIn version 3.3
<210> 1
   <211> 3033
   <212> DNA
   <213> Phaeodactylum tricornutum
<220>
   <221> CDS
   <222> (1)..(3033)
<400> 1
   atg agc ggg gca aat tat aga gaa gcc gac ttc cct ggt gtc cgt gcc      48
   Met Ser Gly Ala Asn Tyr Arg Glu Ala Asp Phe Pro Gly Val Arg Ala
   1               5                   10                  15
   gcg ggt cgg cac aac aat tcc att aca acc aaa gag ctg acg gaa tgt      96
   Ala Gly Arg His Asn Asn Ser Ile Thr Thr Lys Glu Leu Thr Glu Cys
               20                  25                  30
   gat cgt gag cct gtg cac ttg atc gca aac gta caa ggg ggt acc ggc     144
   Asp Arg Glu Pro Val His Leu Ile Ala Asn Val Gln Gly Gly Thr Gly
           35                  40                  45
   cat ttg ttg ttc att cac tac ccg tct gga aaa atc ttg gct cat gat     192
   His Leu Leu Phe Ile His Tyr Pro Ser Gly Lys Ile Leu Ala His Asp
       50                  55                  60
   cgc gac atc gaa cac att cct tgg atc cgg tgt cac gaa aac aga aca     240
   Arg Asp Ile Glu His Ile Pro Trp Ile Arg Cys His Glu Asn Arg Thr
   65                  70                  75                  80
   gtt acc gct ggc cgc act ggg gcg aga act aca tct tct ttc cat gga     288
   Val Thr Ala Gly Arg Thr Gly Ala Arg Thr Thr Ser Ser Phe His Gly
                   85                  90                  95
   gaa cag cag agt ggt gag agt cct cac gaa gct att ggc ata tct gga     336
   Glu Gln Gln Ser Gly Glu Ser Pro His Glu Ala Ile Gly Ile Ser Gly
               100                 105                 110
   ggc ttt tta ctg aac tgg gtt ccg cac gat ttc tac gag aag att ctc     384
   Gly Phe Leu Leu Asn Trp Val Pro His Asp Phe Tyr Glu Lys Ile Leu
           115                 120                 125
   gat ttg gtc ctc ggt att atc cat tcc gat acg cac aga aat ttt tat     432
   Asp Leu Val Leu Gly Ile Ile His Ser Asp Thr His Arg Asn Phe Tyr
       130                 135                 140
   ttt tat tca tat gat ggt tca gcg tac gct att tct att tca gcg acg     480
   Phe Tyr Ser Tyr Asp Gly Ser Ala Tyr Ala Ile Ser Ile Ser Ala Thr
   145                 150                 155                 160
   gaa atg gac tac tcc gtg att ggc atc gaa att gaa aca gtt ggt ttg     528
   Glu Met Asp Tyr Ser Val Ile Gly Ile Glu Ile Glu Thr Val Gly Leu
                   165                 170                 175
   gat gat act gcc tcc cat ttt tca tcc tca ttg ttg cat ttg gga cgt     576
   Asp Asp Thr Ala Ser His Phe Ser Ser Ser Leu Leu His Leu Gly Arg
               180                 185                 190
   att gtg gaa ttc tac cag cac gaa gca att gcc aag aca gcc tgt gac       624
   Ile Val Glu Phe Tyr Gln His Glu Ala Ile Ala Lys Thr Ala Cys Asp
           195                 200                 205
   act gtt ttt cac cta ttg gga aag tat gac agg ggc atg gtg tac cga       672
   Thr Val Phe His Leu Leu Gly Lys Tyr Asp Arg Gly Met Val Tyr Arg
       210                 215                 220
   ttc cac gat gat ctg tcc ggc gag gtc gtg cac gag att aaa gca aat       720
   Phe His Asp Asp Leu Ser Gly Glu Val Val His Glu Ile Lys Ala Asn
   225                 230                 235                 240
   cat gtg gaa tcc agc tat ctt ggc atg cga ttt cct tcc tct gat att       768
   His Val Glu Ser Ser Tyr Leu Gly Met Arg Phe Pro Ser Ser Asp Ile
                   245                 250                 255
   cct ttg cca tcg cga cag ctt tat ata aaa aat ggt gtg cgg tac att       816
   Pro Leu Pro Ser Arg Gln Leu Tyr Ile Lys Asn Gly Val Arg Tyr Ile
               260                 265                 270
   tac gac gtt gat acc gag gat cta ccg att tta tcc ctg gac aat gaa       864
   Tyr Asp Val Asp Thr Glu Asp Leu Pro Ile Leu Ser Leu Asp Asn Glu
           275                 280                 285
   aag atg gat ctc agt caa att cgc atg cgt gct gta gcc aaa ccg cat       912
   Lys Met Asp Leu Ser Gln Ile Arg Met Arg Ala Val Ala Lys Pro His
       290                 295                 300
   att gtg tac tta aga aat atg ggg gtg gtg tcg tcg ttg agc ttg gcg       960
   Ile Val Tyr Leu Arg Asn Met Gly Val Val Ser Ser Leu Ser Leu Ala
   305                 310                 315                 320
   att gtt gtc gac aat gat ctg tgg ggg ttg ctg gct ttt cat ggg tac      1008
   Ile Val Val Asp Asn Asp Leu Trp Gly Leu Leu Ala Phe His Gly Tyr
                   325                 330                 335
   ggc gcg agg tac aag cct tcg ctc cat cag cga att gct tgt gaa acc      1056
   Gly Ala Arg Tyr Lys Pro Ser Leu His Gln Arg Ile Ala Cys Glu Thr
               340                 345                 350
   ata agt gcg atg gtc tca gtt cgt att gaa tct ctc atg aaa aag gcg      1104
   Ile Ser Ala Met Val Ser Val Arg Ile Glu Ser Leu Met Lys Lys Ala
           355                 360                 365
   cag agt gcc cga att att aag ttg ggc cag tgc act atg agc tta aag      1152
   Gln Ser Ala Arg Ile Ile Lys Leu Gly Gln Cys Thr Met Ser Leu Lys
       370                 375                 380
   cat gac cag agc ctg att cac aat ctc tat gaa tgg ggt gaa ggc ata      1200
   His Asp Gln Ser Leu Ile His Asn Leu Tyr Glu Trp Gly Glu Gly Ile
   385                 390                 395                 400
   ctc gaa att gtt gat gga gat gtt ttg gtt gca cat ata caa gat cct      1248
   Leu Glu Ile Val Asp Gly Asp Val Leu Val Ala His Ile Gln Asp Pro
                   405                 410                 415
   aga gat ggc gaa ggc gac aga att gtg ctg ggt gat cct ttg ttg gta      1296
   Arg Asp Gly Glu Gly Asp Arg Ile Val Leu Gly Asp Pro Leu Leu Val
               420                 425                 430
   ccg aag gat tct ttt tgg act aag atg agt tcc tat cag aat cgc gaa      1344
   Pro Lys Asp Ser Phe Trp Thr Lys Met Ser Ser Tyr Gln Asn Arg Glu
           435                 440                 445
   ctc tgt gtc att tca aca cgc aaa gct ctc aca gat atc aaa ttg aca      1392
   Leu Cys Val Ile Ser Thr Arg Lys Ala Leu Thr Asp Ile Lys Leu Thr
       450                 455                 460
   caa gaa gag tgc cca gca agt gga att gta ttt ttc caa gag ggt cgt      1440
   Gln Glu Glu Cys Pro Ala Ser Gly Ile Val Phe Phe Gln Glu Gly Arg
   465                 470                 475                 480
   act cag atc atg att gga cga gca atg cga tcc aaa gat gtc gta tgg      1488
   Thr Gln Ile Met Ile Gly Arg Ala Met Arg Ser Lys Asp Val Val Trp
                   485                 490                 495
   gca ggt aat cct gac gaa cca aaa cta agg att gga gga att ttg aat      1536
   Ala Gly Asn Pro Asp Glu Pro Lys Leu Arg Ile Gly Gly Ile Leu Asn
               500                 505                 510
   ccg cgc aac tcc ttt act caa ttc att gaa aaa gcg cga aag gaa tca      1584
   Pro Arg Asn Ser Phe Thr Gln Phe Ile Glu Lys Ala Arg Lys Glu Ser
           515                 520                 525
   cga gcc tgg act gtg caa gat att agt gtg att tct gtg ctt cgt gac      1632
   Arg Ala Trp Thr Val Gln Asp Ile Ser Val Ile Ser Val Leu Arg Asp
       530                 535                 540
   cgt ata tgt gag cat tcg tac gca tac atg atg gga tta ctg aga ggt      1680
   Arg Ile Cys Glu His Ser Tyr Ala Tyr Met Met Gly Leu Leu Arg Gly
   545                 550                 555                 560
   gat att caa gat gca aac cgg aaa tat ttg gcg gca att gac aga gcg      1728
   Asp Ile Gln Asp Ala Asn Arg Lys Tyr Leu Ala Ala Ile Asp Arg Ala
                   565                 570                 575
   cgg gac aat tac gaa ttc ttt gcg cat atg agc cac gaa cta cgg act      1776
   Arg Asp Asn Tyr Glu Phe Phe Ala His Met Ser His Glu Leu Arg Thr
               580                 585                 590
   cct ttc cat ggc gtt atg gga tgc tta agt att ctg cat gag tca att      1824
   Pro Phe His Gly Val Met Gly Cys Leu Ser Ile Leu His Glu Ser Ile
           595                 600                 605
   gaa gat atg cca gca gcg gaa gtc aga gat gtt gtc gat aca gca ata      1872
   Glu Asp Met Pro Ala Ala Glu Val Arg Asp Val Val Asp Thr Ala Ile
       610                 615                 620
   gct tcc gga aac cac atg atc aat ctt ctc aac gat att ctg gac atc      1920
   Ala Ser Gly Asn His Met Ile Asn Leu Leu Asn Asp Ile Leu Asp Ile
   625                 630                 635                 640
   tcg aag aac aaa cac ttg tct cat ata tcg gcg cag gat aag gtt att      1968
   Ser Lys Asn Lys His Leu Ser His Ile Ser Ala Gln Asp Lys Val Ile
                   645                 650                 655
   tac cag act tta gcc ttt gag aca att gac tgt atg aag tca ctg gcc      2016
   Tyr Gln Thr Leu Ala Phe Glu Thr Ile Asp Cys Met Lys Ser Leu Ala
               660                 665                 670
   acg tcg cga aag atc gag atg aga tcg tca atc gag ccg aaa ggt ttg      2064
   Thr Ser Arg Lys Ile Glu Met Arg Ser Ser Ile Glu Pro Lys Gly Leu
           675                 680                 685
   gaa aaa gtg gtg att gtg acg gat cgt aca aaa att att caa atc gtt      2112
   Glu Lys Val Val Ile Val Thr Asp Arg Thr Lys Ile Ile Gln Ile Val
       690                 695                 700
   tcc aac gtt gtg aac aat gcc atc aag ttt acg ggt gaa ggg act gtc      2160
   Ser Asn Val Val Asn Asn Ala Ile Lys Phe Thr Gly Glu Gly Thr Val
   705                 710                 715                 720
   gat gtt gta ttt agg ctc gtt gat tcg ctg caa gag gca act atg atg      2208
   Asp Val Val Phe Arg Leu Val Asp Ser Leu Gln Glu Ala Thr Met Met
                   725                 730                 735
   tgg gag cga ggc gcg gaa gtt cat gct gga tca gtg ttt tcg atg aag      2256
   Trp Glu Arg Gly Ala Glu Val His Ala Gly Ser Val Phe Ser Met Lys
               740                 745                 750
   gag agt gaa atg cac aca tcg gct gaa gaa gta aga cgg agc acc atg      2304
   Glu Ser Glu Met His Thr Ser Ala Glu Glu Val Arg Arg Ser Thr Met
           755                 760                 765
   acg ttt aat gag acg cat gat caa aag tgg atg aca atg agt gtc tca      2352
   Thr Phe Asn Glu Thr His Asp Gln Lys Trp Met Thr Met Ser Val Ser
       770                 775                 780
   gac acc gga tgc ggt atg gag ccg tct gaa cta gta gaa atg ttc tca      2400
   Asp Thr Gly Cys Gly Met Glu Pro Ser Glu Leu Val Glu Met Phe Ser
   785                 790                 795                 800
   cca tat acc caa tcg agt cat gga tcc aat cgc att ttt cag gga aca      2448
   Pro Tyr Thr Gln Ser Ser His Gly Ser Asn Arg Ile Phe Gln Gly Thr
                   805                 810                 815
   ggg ctt ggg ctt ttc att tgc gtt tca tta tgt tac caa ctc aat ggt      2496
   Gly Leu Gly Leu Phe Ile Cys Val Ser Leu Cys Tyr Gln Leu Asn Gly
               820                 825                 830
   ttt att tct tgt gcg agc acc ccc gat aaa gga aca ctt ttt cat atg      2544
   Phe Ile Ser Cys Ala Ser Thr Pro Asp Lys Gly Thr Leu Phe His Met
           835                 840                 845
   gga atc cca gtc gga ttg tta gct gaa gac aca gtt gag gga aat cag      2592
   Gly Ile Pro Val Gly Leu Leu Ala Glu Asp Thr Val Glu Gly Asn Gln
       850                 855                 860
   aca cta aca gat gat acg aag gaa aca gag agc gtg atc caa atg tcg      2640
   Thr Leu Thr Asp Asp Thr Lys Glu Thr Glu Ser Val Ile Gln Met Ser
   865                 870                 875                 880
   ggt ccg att ttg atc gta gat gac aat gtt gtg aac gtg aaa att cta      2688
   Gly Pro Ile Leu Ile Val Asp Asp Asn Val Val Asn Val Lys Ile Leu
                   885                 890                 895
   aac cgg gcg cta ctt ttg gat att aga aga gct ggt ctt gca ata gag      2736
   Asn Arg Ala Leu Leu Leu Asp Ile Arg Arg Ala Gly Leu Ala Ile Glu
               900                 905                 910
   gtc ctc aca gca ggg ggt ggg gct gaa ggt gtc cag gtc ttt cga gac      2784
   Val Leu Thr Ala Gly Gly Gly Ala Glu Gly Val Gln Val Phe Arg Asp
           915                 920                 925
   aag cgc ccc agt cta tgc att atc gac tat cac atg ccc gat gtc gat      2832
   Lys Arg Pro Ser Leu Cys Ile Ile Asp Tyr His Met Pro Asp Val Asp
       930                 935                 940
   ggc att gaa gcg acc tgc acc ata cgg aaa tac gag caa gaa aac aaa      2880
   Gly Ile Glu Ala Thr Cys Thr Ile Arg Lys Tyr Glu Gln Glu Asn Lys
   945                 950                 955                 960
   att gat cct acc tac att ttg atg tac act gct gat gcc aca gag caa      2928
   Ile Asp Pro Thr Tyr Ile Leu Met Tyr Thr Ala Asp Ala Thr Glu Gln
                   965                 970                 975
   gct aga gca ttg atc ttg agc tcc ggc gtt gac gat atc atg tcc aag      2976
   Ala Arg Ala Leu Ile Leu Ser Ser Gly Val Asp Asp Ile Met Ser Lys
               980                 985                 990
   cct ccg ccg aag gga ttc att gcc  ggg ttg gtg cag agg  ctg cgg gtt    3024
   Pro Pro Pro Lys Gly Phe Ile Ala  Gly Leu Val Gln Arg  Leu Arg Val
           995                 1000                 1005
   ccg gaa  tag                                                         3033
   Pro Glu
       1010
<210> 2
   <211> 1010
   <212> PRT
   <213> Phaeodactylum tricornutum
<400> 2
   Met Ser Gly Ala Asn Tyr Arg Glu Ala Asp Phe Pro Gly Val Arg Ala
   1               5                   10                  15
   Ala Gly Arg His Asn Asn Ser Ile Thr Thr Lys Glu Leu Thr Glu Cys
               20                  25                  30
   Asp Arg Glu Pro Val His Leu Ile Ala Asn Val Gln Gly Gly Thr Gly
           35                  40                  45
   His Leu Leu Phe Ile His Tyr Pro Ser Gly Lys Ile Leu Ala His Asp
       50                  55                  60
   Arg Asp Ile Glu His Ile Pro Trp Ile Arg Cys His Glu Asn Arg Thr
   65                  70                  75                  80
   Val Thr Ala Gly Arg Thr Gly Ala Arg Thr Thr Ser Ser Phe His Gly
                   85                  90                  95
   Glu Gln Gln Ser Gly Glu Ser Pro His Glu Ala Ile Gly Ile Ser Gly
               100                 105                 110
   Gly Phe Leu Leu Asn Trp Val Pro His Asp Phe Tyr Glu Lys Ile Leu
           115                 120                 125
   Asp Leu Val Leu Gly Ile Ile His Ser Asp Thr His Arg Asn Phe Tyr
       130                 135                 140
   Phe Tyr Ser Tyr Asp Gly Ser Ala Tyr Ala Ile Ser Ile Ser Ala Thr
   145                 150                 155                 160
   Glu Met Asp Tyr Ser Val Ile Gly Ile Glu Ile Glu Thr Val Gly Leu
                   165                 170                 175
   Asp Asp Thr Ala Ser His Phe Ser Ser Ser Leu Leu His Leu Gly Arg
               180                 185                 190
   Ile Val Glu Phe Tyr Gln His Glu Ala Ile Ala Lys Thr Ala Cys Asp
           195                 200                 205
   Thr Val Phe His Leu Leu Gly Lys Tyr Asp Arg Gly Met Val Tyr Arg
       210                 215                 220
   Phe His Asp Asp Leu Ser Gly Glu Val Val His Glu Ile Lys Ala Asn
   225                 230                 235                 240
   His Val Glu Ser Ser Tyr Leu Gly Met Arg Phe Pro Ser Ser Asp Ile
                   245                 250                 255
   Pro Leu Pro Ser Arg Gln Leu Tyr Ile Lys Asn Gly Val Arg Tyr Ile
               260                 265                 270
   Tyr Asp Val Asp Thr Glu Asp Leu Pro Ile Leu Ser Leu Asp Asn Glu
           275                 280                 285
   Lys Met Asp Leu Ser Gln Ile Arg Met Arg Ala Val Ala Lys Pro His
       290                 295                 300
   Ile Val Tyr Leu Arg Asn Met Gly Val Val Ser Ser Leu Ser Leu Ala
   305                 310                 315                 320
   Ile Val Val Asp Asn Asp Leu Trp Gly Leu Leu Ala Phe His Gly Tyr
                   325                 330                 335
   Gly Ala Arg Tyr Lys Pro Ser Leu His Gln Arg Ile Ala Cys Glu Thr
               340                 345                 350
   Ile Ser Ala Met Val Ser Val Arg Ile Glu Ser Leu Met Lys Lys Ala
           355                 360                 365
   Gln Ser Ala Arg Ile Ile Lys Leu Gly Gln Cys Thr Met Ser Leu Lys
       370                 375                 380
   His Asp Gln Ser Leu Ile His Asn Leu Tyr Glu Trp Gly Glu Gly Ile
   385                 390                 395                 400
   Leu Glu Ile Val Asp Gly Asp Val Leu Val Ala His Ile Gln Asp Pro
                   405                 410                 415
   Arg Asp Gly Glu Gly Asp Arg Ile Val Leu Gly Asp Pro Leu Leu Val
               420                 425                 430
   Pro Lys Asp Ser Phe Trp Thr Lys Met Ser Ser Tyr Gln Asn Arg Glu
           435                 440                 445
   Leu Cys Val Ile Ser Thr Arg Lys Ala Leu Thr Asp Ile Lys Leu Thr
       450                 455                 460
   Gln Glu Glu Cys Pro Ala Ser Gly Ile Val Phe Phe Gln Glu Gly Arg
   465                 470                 475                 480
   Thr Gln Ile Met Ile Gly Arg Ala Met Arg Ser Lys Asp Val Val Trp
                   485                 490                 495
   Ala Gly Asn Pro Asp Glu Pro Lys Leu Arg Ile Gly Gly Ile Leu Asn
               500                 505                 510
   Pro Arg Asn Ser Phe Thr Gln Phe Ile Glu Lys Ala Arg Lys Glu Ser
           515                 520                 525
   Arg Ala Trp Thr Val Gln Asp Ile Ser Val Ile Ser Val Leu Arg Asp
       530                 535                 540
   Arg Ile Cys Glu His Ser Tyr Ala Tyr Met Met Gly Leu Leu Arg Gly
   545                 550                 555                 560
   Asp Ile Gln Asp Ala Asn Arg Lys Tyr Leu Ala Ala Ile Asp Arg Ala
                   565                 570                 575
   Arg Asp Asn Tyr Glu Phe Phe Ala His Met Ser His Glu Leu Arg Thr
               580                 585                 590
   Pro Phe His Gly Val Met Gly Cys Leu Ser Ile Leu His Glu Ser Ile
           595                 600                 605
   Glu Asp Met Pro Ala Ala Glu Val Arg Asp Val Val Asp Thr Ala Ile
       610                 615                 620
   Ala Ser Gly Asn His Met Ile Asn Leu Leu Asn Asp Ile Leu Asp Ile
   625                 630                 635                 640
   Ser Lys Asn Lys His Leu Ser His Ile Ser Ala Gln Asp Lys Val Ile
                   645                 650                 655
   Tyr Gln Thr Leu Ala Phe Glu Thr Ile Asp Cys Met Lys Ser Leu Ala
               660                 665                 670
   Thr Ser Arg Lys Ile Glu Met Arg Ser Ser Ile Glu Pro Lys Gly Leu
           675                 680                 685
   Glu Lys Val Val Ile Val Thr Asp Arg Thr Lys Ile Ile Gln Ile Val
       690                 695                 700
   Ser Asn Val Val Asn Asn Ala Ile Lys Phe Thr Gly Glu Gly Thr Val
   705                 710                 715                 720
   Asp Val Val Phe Arg Leu Val Asp Ser Leu Gln Glu Ala Thr Met Met
                   725                 730                 735
   Trp Glu Arg Gly Ala Glu Val His Ala Gly Ser Val Phe Ser Met Lys
               740                 745                 750
   Glu Ser Glu Met His Thr Ser Ala Glu Glu Val Arg Arg Ser Thr Met
           755                 760                 765
   Thr Phe Asn Glu Thr His Asp Gln Lys Trp Met Thr Met Ser Val Ser
       770                 775                 780
   Asp Thr Gly Cys Gly Met Glu Pro Ser Glu Leu Val Glu Met Phe Ser
   785                 790                 795                 800
   Pro Tyr Thr Gln Ser Ser His Gly Ser Asn Arg Ile Phe Gln Gly Thr
                   805                 810                 815
   Gly Leu Gly Leu Phe Ile Cys Val Ser Leu Cys Tyr Gln Leu Asn Gly
               820                 825                 830
   Phe Ile Ser Cys Ala Ser Thr Pro Asp Lys Gly Thr Leu Phe His Met
           835                 840                 845
   Gly Ile Pro Val Gly Leu Leu Ala Glu Asp Thr Val Glu Gly Asn Gln
       850                 855                 860
   Thr Leu Thr Asp Asp Thr Lys Glu Thr Glu Ser Val Ile Gln Met Ser
   865                 870                 875                 880
   Gly Pro Ile Leu Ile Val Asp Asp Asn Val Val Asn Val Lys Ile Leu
                   885                 890                 895
   Asn Arg Ala Leu Leu Leu Asp Ile Arg Arg Ala Gly Leu Ala Ile Glu
               900                 905                 910
   Val Leu Thr Ala Gly Gly Gly Ala Glu Gly Val Gln Val Phe Arg Asp
           915                 920                 925
   Lys Arg Pro Ser Leu Cys Ile Ile Asp Tyr His Met Pro Asp Val Asp
       930                 935                 940
   Gly Ile Glu Ala Thr Cys Thr Ile Arg Lys Tyr Glu Gln Glu Asn Lys
   945                 950                 955                 960
   Ile Asp Pro Thr Tyr Ile Leu Met Tyr Thr Ala Asp Ala Thr Glu Gln
                   965                 970                 975
   Ala Arg Ala Leu Ile Leu Ser Ser Gly Val Asp Asp Ile Met Ser Lys
               980                 985                 990
   Pro Pro Pro Lys Gly Phe Ile Ala  Gly Leu Val Gln Arg  Leu Arg Val
           995                 1000                 1005
   Pro Glu
       1010
<210> 3
   <211> 25
   <212> DNA
   <213> Artificial Sequence
<220>
   <223> synthetic primer
<400> 3
   actgaatcca gctatcttgg catgc 25
<210> 4
   <211> 26
   <212> DNA
   <213> Artificial Sequence
<220>
   <223> synthetic primer
<400> 4
   acttctagat cattgtcgac aacaat 26
<210> 5
   <211> 26
   <212> DNA
   <213> Artificial Sequence
<220>
   <223> synthetic primer
<400> 5
   gattctagag ctcatagtgc actggc 26
<210> 6
   <211> 26
   <212> DNA
   <213> Artificial Sequence
<220>
   <223> synthetic primer
<400> 6
   agtccgcgga atctcgccta ttcatg 26
<210> 7
   <211> 20
   <212> DNA
   <213> Artificial Sequence
<220>
   <223> synthetic primer
<400> 7
   gcgtacgctg aaccatcata 20
<210> 8
   <211> 20
   <212> DNA
   <213> Artificial Sequence
<220>
   <223> synthetic primer
<400> 8
   gttccgcacg atttctacga 20
<210> 9
   <211> 20
   <212> DNA
   <213> Artificial Sequence
<220>
   <223> synthetic primer
<400> 9 20
   gaattgtgct gggtgatcct 20
<210> 10
   <211> 20
   <212> DNA
   <213> Artificial Sequence
<220>
   <223> synthetic primer
<400> 10
   gtgagagctt tgcgtgttga 20
<210> 11
   <211> 20
   <212> DNA
   <213> Artificial Sequence
<220>
   <223> synthetic primer
<400> 11
   gacatcgggc atgtgatagt 20
<210> 12
   <211> 21
   <212> DNA
   <213> Artificial Sequence
<220>
   <223> synthetic primer
<400> 12
   gcaatagagg tcctcacagc a 21
<210> 13
   <211> 21
   <212> DNA
   <213> Artificial Sequence
<220>
   <223> synthetic primer
<400> 13
   gtgcaagaga ccggacatac c 21
<210> 14
   <211> 21
   <212> DNA
   <213> Artificial Sequence
<220>
   <223> synthetic primer
<400> 14
   cgaagtcaac caggaaacca a 21
<210> 15
   <211> 437
   <212> DNA
   <213> Phaeodactylum tricornutum
<400> 15
   aatctcgcct attcatggtg tataaaagtt caacatccaa agctagaact tttggaaaga     60
   gaaagaatat ccgaataggg cacggcgtgc cgtattgttg gagtggacta gcagaaagtg    120
   aggaaggcac aggatgagtt ttctcgagac acatagcttc agcgtcgtgt aggctaggca    180
   gaggtgagtt ttctcgagac ataccttcag cgtcgtcttc actgtcacag tcaactgaca    240
   gtaatcgttg atccggagag attcaaaatt caatctgttt ggacctggat aagacacaag    300
   agcgacatcc tgacatgaac gccgtaaaca gcaaatcctg gttgaacacg tatccttttg    360
   ggggcctccg ctacgacgct cgctccagct ggggcttcct tactatacac agcgcgcata    420
   tttcacggtt gccagaa                                                   437
<210> 16
   <211> 650
   <212> DNA
   <213> Phaeodactylum tricornutum
<400> 16
   gcaatctcac gcaccaggcg ctggaagggc aacttgcgga tgagaaggtc cgtggacttc     60
   tggtaacgac ggatctcacg cagagcgacg gttccagggc gataacggtg gggcttcttg    120
   actcctccgg tagccggagc ggacttgcgg gcagccttgg tggcaagctg cttgcgcggc    180
   gctttgcctc cggtggattt acgggcggtt tgcttggttc gggccatttt gacggttttt    240
   tttacaagag aagagttctt gaaatttgtg aggttaaagt gtgtggcttc cgccgtagtc    300
   aaggagcgtg cggttgccga tcgcaccggt acgttctgta gaaatgaaca cagtgtgttg    360
   aattgaaagt atggcgcagg tatggtgtgt gataagtagc agccgcgccg agacaaacaa    420
   actttggttt ctacgacaat ctctgtagac aagtactaga aacccgtttg aacgagcata    480
   aatctgcacc ggcaggccac cagacatcgt ttcaacgtaa tattctacgt aaccatttta    540
   tcccaggaaa cctacggcct gtgaaccacc gagacggagc actcacaatt cgctctcggc    600
   aacaaccgac aatcgtctta ctcacagtca ataccgaaaa caaacaacag               650
<210> 17
   <211> 953
   <212> PRT
   <213> Thalassiosira pseudonana
<400> 17
   Met Ile Gly Lys Pro Leu Gly Leu Ile Phe Gly Ala Glu His Val Ser
   1               5                   10                  15
   Gln Val Lys Asp Val Ile Glu Asn Val Ile Met Thr Ser Gly Gly Gly
               20                  25                  30
   Gly Gly Ser Ala Ser Ser Ser Gly Glu Arg His Ser Leu Pro Ser Phe
           35                  40                  45
   Ile Gln Pro Pro Ser Lys Arg Arg Val Ser Asp Asp Asn Phe Leu Leu
       50                  55                  60
   Arg Ser Glu Ser Thr Leu Ser Leu Ser Gly Arg Ile Ser Cys Ser Val
   65                  70                  75                  80
   Leu Pro Ser Ser Gln Asn Phe Leu Leu Glu Leu Glu Lys Thr Pro Leu
                   85                  90                  95
   Leu Glu Lys Tyr Thr Gln Phe Glu Asp Arg Asp Val Met Ser Phe Met
               100                 105                 110
   Glu Glu Ile Ala Lys Glu Leu Arg Ala Cys Trp Ser Ile Glu Glu Met
           115                 120                 125
   Ala Ser Leu Val Cys Ala Lys Val Met Gln Glu Thr Pro Tyr Asp Arg
       130                 135                 140
   Gly Met Val Tyr Lys Phe Asp His Glu Asp Cys Gly Glu Val Val Tyr
   145                 150                 155                 160
   Glu Ala Phe Arg Ser Asp Ala Ser Glu Ala Cys Arg Lys Asp Ser Phe
                   165                 170                 175
   Leu Gly Leu Arg Phe Pro Ala Ser Asp Ile Pro Arg Gln Ala Arg Glu
               180                 185                 190
   Leu Phe Met Arg Asn Thr Leu Arg Val Val Tyr Asp Val Asp Gly Asn
           195                 200                 205
   Asp Phe Glu Leu Tyr Pro Pro Met Val Asp Ile Lys Arg Ala Ser Gly
       210                 215                 220
   Glu Lys Glu Leu Gly Tyr Thr Asp Leu Ser Met Cys Arg Leu Arg Gly
   225                 230                 235                 240
   Ser Ser Phe Val His Leu Lys Tyr Leu Lys Asn Met Gly Val Thr Ser
                   245                 250                 255
   Thr Met Val Ile Ala Ile Ile Val Asn Gly Arg Leu Trp Gly Leu Tyr
               260                 265                 270
   Ser Phe His Gly Tyr Arg Glu Pro Leu Val Pro Ser Ala Arg Thr Arg
           275                 280                 285
   Phe Leu Cys Glu Met Ala Ser Ile Thr Thr Ser Ile Ile Met Glu Ser
       290                 295                 300
   Leu Thr Arg Lys Glu Ser Asn Glu Arg Leu Met Ser Leu Asp Ser Leu
   305                 310                 315                 320
   Met Asn Ser Leu Gln Thr Thr Ser Leu Ser Asn Phe Val Glu Thr Asn
                   325                 330                 335
   Leu Ser Asp Ile Met Gly Ala Leu Gln Val Asn Leu Ile Ser Phe Arg
   340 345 350
   Val Arg Asp Pro Pro Ala Thr Pro Glu Ile Lys Thr Phe Leu Asp Glu
   355 360 365
   Thr Asp Gly Lys Met Glu Ser Pro Asp Glu Ile Thr Asn Glu Val Phe
   370 375 380
   Asp Ser Leu Val Glu Thr Tyr Gly Ser Val Cys Arg Asp Tyr Gly Val
   385 390 395 400
   Val Tyr Ile Asp Asp Gln Lys Ser Asn Thr Leu Leu Val Asn Arg Gly
   405 410 415
   Ile His Thr Leu Val Phe Phe Arg Thr Ser Gly Val Asp Val Ile Leu
   420 425 430
   Ser Arg Asn Arg Thr Ile Glu Arg Val Thr Trp Gly Gly Asp Pro Asp
   435 440 445
   Lys Gln Leu Glu Pro Asp Gly Thr Leu Thr Pro Arg Asn Ser Phe Ala
   450 455 460
   Ala Tyr Val Glu Asp His Phe Lys Lys Gly Lys Pro Trp Asp Ala Thr
   465 470 475 480
   Asp Arg Gln Leu Ile Ser Arg Phe Ser Asp Gln Leu Glu Lys His Arg
   485 490 495
   Thr Lys Glu Leu Asn Leu Glu His Ser Lys Thr Ile Lys Thr Leu Glu
   500 505 510
   Lys Glu Lys Ala Glu Ala Thr Glu Thr Ala Arg Val Asn Phe Asp Phe
   515 520 525
   Phe Ala His Met Ala His Glu Leu Arg Thr Pro Phe His Gly Met Val
   530 535 540
   Gly Ser Leu Glu Ala Met Arg Glu Asp Pro Val Leu Arg Asn Asn Glu
   545 550 555 560
   Met Leu Lys Thr Ala Glu Leu Cys Gly Lys Ser Met Ile Lys Ile Leu
   565 570 575
   Asp Asp Ile Leu Leu Val Ala Lys Gly Ser Tyr Asp Leu Gln Ile Glu
   580 585 590
   Glu His Leu Phe Asp Leu Lys Ser Phe Val Lys Ile Thr Ala Asn Asp
   595 600 605
   Met Ser Ser Phe Ala Leu Met Glu Gly Ala Pro Ile Gln Leu Gly Lys
       610                 615                 620
   Glu Leu Ile Phe Cys Lys Asp Leu Val Gly Asp Ser Gln Arg Ile Arg
   625                 630                 635                 640
   Gln Val Met Asn Asn Leu Leu Ser Asn Ala Ile Lys Phe Ser Glu Asp
                   645                 650                 655
   Ser Ile Ser Leu Asp Val Val Gln Lys Asn Ser Phe Ser Glu Val Leu
               660                 665                 670
   Ala Val Trp Lys Thr Tyr Ser Asn Val Tyr Pro Asn Cys Glu Pro Thr
           675                 680                 685
   Leu Leu Glu Leu Ser Thr Phe Val Ala Glu Ala Ser Glu Ala Ser Val
       690                 695                 700
   Asp Thr Ile Trp Val Ile Phe Ser Ile Val Asp Lys Gly Ile Gly Ile
   705                 710                 715                 720
   Thr Gly Pro Asp Leu Lys Lys Leu Gly Thr Ala Phe Thr Gln Leu Ser
                   725                 730                 735
   Ser Gly Arg Gln Lys Lys Tyr Gln Gly Thr Gly Leu Gly Leu Asn Ile
               740                 745                 750
   Cys Asn Met Leu Val Gly Ala Leu Gly Gly Lys Leu Val Met Phe Ser
           755                 760                 765
   Ala Lys Gly Leu Gly Ser Cys Phe Thr Phe Ala Val Pro Val Lys Lys
       770                 775                 780
   Ser Asp Val Pro Val Ala Glu Pro Glu Val Glu Glu Ser Gln Asn Glu
   785                 790                 795                 800
   Lys Ala Lys Lys Met Glu Ala Leu Gln Glu Glu Phe Asp Ala Phe Gly
                   805                 810                 815
   Phe Arg Glu Lys Gly Val Arg Ile Phe Val Val Asp Asp Ser Ala Ile
               820                 825                 830
   Asn Arg Lys Leu Cys Gly Arg Lys Ile Arg Arg Trp Met Pro Ser Val
           835                 840                 845
   Thr Ile Lys Glu Cys Ser Ser Gly Ser Ser Ala Leu Ala Glu Tyr Glu
       850                 855                 860
   Lys Ser Pro Lys Asp Val Met Gly Ile Phe Met Asp Tyr His Met Gln
   865                 870                 875                 880
   Asp Met Asp Gly Asp Glu Cys Thr Arg Arg Ile Arg Glu Cys Glu Ser
                   885                 890                 895
   Met Arg Glu Asp Leu Pro Asn Val Tyr Ile Val Gly Tyr Thr Ala Asp
               900                 905                 910
   Val Leu Glu Asp Ser Thr Gly Arg Leu Met Glu Ser Gly Met Asn Ser
           915                 920                 925
   Val Met Pro Lys Pro Glu Pro Pro Arg Gly Glu Cys Asp Ser Lys Leu
       930                 935                 940
   Ser Ile Arg Lys Thr Ser Arg Pro Leu
   945                 950

## Description

### FIELD OF THE INVENTION

The present invention relates to a method to induce alga and/or diatom cell spontaneous flocculation comprising inactivating the expression of a photoreceptor in said alga and/or diatom, to constructs able to inhibit the expression of the photoreceptor and uses thereof.

### BACKGROUND ART

Diatoms are unicellular photosynthetic eukaryotes found in most humid environments and open water masses that play a major role in the global cycling of carbon and silicon (Falkowski, 1998). They are thought to have arisen from a secondary endosymbiotic event between three eukaryotes, a red alga, a green alga, and a flagellated heterotroph. Recent analysis of diatom genomes indeed reveals a 'mosaic' nature, with genes derived from plant, animal and bacterial lineages (Armbrust, et al., 2004, Bowler, et al., 2008). Diatom cells therefore have a range of features that make them highly divergent from the classical cellular structures of higher plants and animals, so studies of diatom biology promise to reveal many novel aspects of commercial interest. Basic studies of diatom cell biology have nonetheless been hampered in the past by the lack of a model species and associated molecular tools. This situation is now changing following the emergence of two species, *Thalassiosira pseudonana* and *Phaeodactylum tricornutum,* that are finally beginning to yield the molecular secrets of diatoms (Armbrust, et al., 2004, Bowler, et al., 2008). Recent advances in molecular genomics, such as the development of generic Gateway-based transformation vectors for overexpression (Siaut et al., 2007) and RNAi-based gene knockdown approaches (De Riso et al., 2009) can also facilitate the use of diatom specific genes or pathways for biotechnology. Techniques to grow *P. tricornutum* heterotrophically have also been developed and pave the way for large-scale cultivation using microbial fermentation technology (Zaslavskaia, et al., 2001).

Biologically active compounds extracted from diatom cells have been proposed for a range of biotechnological applications (for a review see Lebeau and Robert 2003). Some diatoms synthesize the nutritionally important eicosapentaenoic (EPA) and docosahexaenoic (DHA) acids as well. PUFAs (e.g., polyunstaurated fatty acids, PUFAs) generally constitute around 25% of the total synthesized fatty acids in diatoms (Dunstan et al. 1994), and the total content can be as high as 50% of total biomass (Chisti 2007; 2008). Because some diatoms can now be genetically modified, it is possible that strains capable of synthesizing higher levels of specific PUFAs can be generated. The production of commercially desirable PUFAs could be significantly enhanced using such methods. Furthermore, diatoms are nutritionally suitable as feedstock for the mariculture industry, and they may be useful for the bioremediation of water contaminated with heavy metals or enriched in phosphate and nitrogen.

Because of their high lipid content and fatty acid composition, diatoms are also a promising source of biofuels. In fact oil productivity in many diatoms greatly exceeds the oil productivity of the best oil producing crops, making them interesting for commercial exploitation (Carrieri et al. 2008; Dismukes et al. 2008). Moreover the biotechnological exploitation of diatoms has several advantages over plants. Diatoms are able to grow at high rates thanks to their nutrient uptake system and can grow on waste, brackish, and sea water. Furthermore, biomass yields are five to ten times higher than plants because the turnover times of diatom cells are much faster, and energy yields can be between six and twelve times higher (up to 60 T/ha). Additionally, because they do not produce lignin or cellulose, as plants do, the non-lipid fraction can be used as a high protein feed in the livestock and poultry industries.

Several factors have impeded up to now the exploitation of diatoms for large scale commercial applications (Chisti 2007; 2008). In addition to the lack of efforts devoted to developing the appropriate engineering technologies, the strains that could be used are essentially wild type strains that have not been optimized for lipid production. Natural populations therefore need to be screened for high oil production and appropriate strains need to undergo mutagenesis or genetic engineering programs to improve their suitability for commercial scale production.

Genetic and metabolic engineering are likely to have the greatest impact on improving the economics of diatom biofuel production (Chisti 2007, 2008). Molecular level engineering can be used to:
1. increase photosynthetic efficiency to enable increased biomass;
2. enhance biomass growth rate;
3. increase oil content of biomass;
4. improve temperature tolerance to reduce the expense of cooling;
5. extend photosynthetic yields and reduce photoinhibition at higher light intensities;
6. identify factors that stimulate oil production;

In addition, all the production systems are needed to optimize harvesting of cells for the subsequent recovery of products/molecules of interest.

The basic concept of extensive microalgal exploitation in biotechnology is the use of relevant biomass that would need to be concentrated and recovered from the growth medium to allow for the ensuing industrial treatments. The biomass must be generally concentrated by an initial factor of at least about thirty-fold, requiring, for convenient industrial application, very low-cost harvesting processes such as "bioflocculation", or "flocculation", namely a spontaneous flocculation-sedimentation process of the algal cells, using no, or at most very little, flocculation chemicals. In fact, for the selection of the algal strains to cultivate the harvestability is one of the primary criteria. Up to now this step is very critical in the case of diatom macrocultures and the development of low-cost harvesting processes is a very relevant industrial goal to be realised. The controlled induction of the diatom cell autoaggregation-sedimentation, proposed in the present document is based on the inhibition of photoreceptor activity, which causes flocculation. A specific example is provided using phytochrome inactivated using an antisense strategy, alternatively inverted repeat fragment based inactivation may be used, representing a very low-cost harvesting process of interest to several industrial fields.

### SUMMARY OF THE INVENTION

In the present invention it was found that it is possible to silence the *PHY* gene by a construct comprising either antisense or inverted repeat fragments of the *PHY* gene. The transcription of such silencing constructs can be driven by various promoters, such as for example FcpBp or H4p. The introduction of the silencing constructs in *P. tricornutum* leads to a decrease in protein expression, preferably between 50 and 90 %.

Surprisingly such cells do flocculate spontaneously very efficiently. In the present invention terms as flocculation, sedimentation, bioflocculation or aggregation are used and mean a process method whereby cells come out of suspension by means of a specific altered genotype/phenotype, i.e. because phytochrome inactivation causes the cells to aggregate and sediment. Using this method, cultured cells can be harvested easily, without addition of chemicals.

It is therefore an object of the invention a method to induce alga and/or diatom cell bio-flocculation comprising the step of inactivating the expression of at least one photoreceptor in said alga and/or diatom, wherein the photoreceptor is a phytochrome. Preferably the alga is selected from any of the following groups: the species Chromalveolata and Archaeplastida or the group of Bacillariophyceae; alternatively the diatom is selected from the species Thalassiosira pseudonana or Phaeodactylum tricornutum.

In a preferred embodiment the phytochrome is the Phy protein, essentially consisting of SEQ ID. No. 2.

In a preferred embodiment the inactivation of the expression of the photoreceptor is achieved by means of genetic engineering, preferably comprising the step of transforming the alga and/or the diatom cell with a nucleic acid construct able to inactivate the expression of the photoreceptor. More preferably the nucleic acid construct able to inactivate the expression of the photoreceptor comprises an alga and/or diatom cell active promoter operatively linked to a nucleic acid fragment of the photoreceptor cDNA. Most preferably the nucleic acid construct is able to inactivate the expression of the photoreceptor Phy protein, essentially consisting of SEQ ID. No. 2, comprising a diatom active promoter region and operatively linked to a nucleic acid fragment of the *PHY* cDNA comprised between nt 727 and nt 980 of SEQ ID No. 1 or between nt 727 and nt 1147 of SEQ ID No. 1 introduced in the antisense orientation with respect to the nucleic acid fragment of the *PHY* cDNA. In a further preferred aspect the promoter region is selected from the promoter region of the Fucoxanthin Chlorophyll a/c-binding Protein B gene (ID number 56299, SEQ ID. No. 15) or of the Histone 4 gene (ID number 34971, SEQ ID. No. 16).

It is another object of the invention the use of an alga and/or diatom cell transformed with a DNA construct able to inactivate the expression of the photoreceptor Phy protein, essentially consisting of SEQ ID. No. 2, comprising a diatom active promoter region and operatively linked to a nucleic acid fragment of the *PHY* cDNA comprised between nt 727 and nt 980 of SEQ ID No. 1 or between nt 727 and nt 1147 of SEQ ID No. 1 introduced in the antisense orientation with respect to the nucleic acid fragment of the *PHY* cDNA, wherein the promoter region is preferably selected from the promoter region of the Fucoxanthin Chlorophyll a/c-binding Protein B gene (ID number 56299, SEQ ID. No. 15) or of the Histone 4 gene (ID number 34971, SEQ ID. No. 16), for the production of biodiesel and/or natural or recombinant molecules, and/or for the bioremediation of contaminated water or water enriched in phosphate and/or nitrogen, and/or as feed for livestock or poultry.

### DETAILED DESCRIPTION OF THE INVENTION

### FIGURE LEGENDS

The present invention will be now illustrated by means of the following non limiting figures.
**Fig. 1** Comparison of Phy domain organization among plants, cyanobacteria, fungi, bacteria and diatoms.
**Fig. 2** Phylogenetic analysis revealing an independent clade for diatom phytochromes (Phy) and brown algal viruses (Montsant et al., 2007).
**Fig. 3** *PHY* cDNA sequence (SEQ ID. No. 1). The region targeted for the gene silencing is reported in grey and the primers used for the amplification of short and long antisense fragments are in bold character and underlined.
**Fig. 4** Alignment of *P. tricornutum* (PtPHY protein) (SEQ ID No. 2) and *T. pseudonana* (TpPHY protein) (SEQ ID No. 17) proteins.
**Fig. 5** Schematic representation of antisense and inverted repeat constructs for PHY silencing.
**Fig. 6** Analysis of Phy protein by immunoblot in independent silenced clones. Phy levels were quantified using a serial dilution of proteins from wild-type cells as standard and the anti-CPF1 antibody was used as loading control (A). Wild types and F-ir20, H-an4 and H-an6 mutants cultures grown under normal condition. Cell sedimentation is evident on the bottom of the flasks in the mutants (B). Optical and electron microscope analysis of silenced lines (C)
**Fig. 7** Sedimentation features of the wild type (Pt1), F-ir20, H-an4 and H-an6, quantified spectrophotometrically, by following the optical density (OD) decrease over the time (up to 2 hours). The slope of the curve indicates the rate of sedimentation, as the O.D672 nm is proportional to the cell in suspension. All the mutants sediment faster than the wild types, with the H-an6 showing the fastest sedimentation rate.

### MATERIALS AND METHODS

### Cell culture

The CCMP632 strain of *Phaeodactylum tricornutum* Bohlin was obtained from the culture collection of the Provasoli-Guillard National Center for Culture of Marine Phytoplancton (Bigelow Laboratory for Ocean Science-Maine, USA). Cultures were grown in f/2 medium (Guillard 1975), incubated at 18 °C under cool white fluorescent lights at ca. 100 µmol·m⁻²·s⁻¹ in a 12h:12h dark-light cycle.

### Antisense and inverted-repeat constructs

The vectors for antisense and inverted repeat constructs were generated using standard molecular cloning procedures (Sambrook et al. 1989).

For generation of the PHY silencing vectors, the GUS antisense and inverted-repeat vectors described by De Riso et al. (2009) were used.

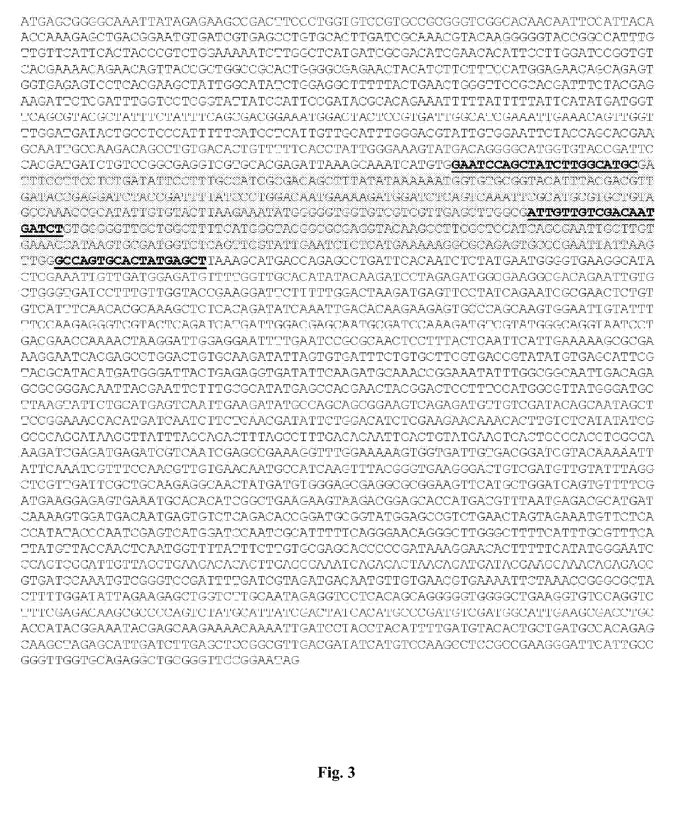

More in detail a 254 bp fragment, corresponding to the *PHY* gene sequence from 727 bp to 980 bp (Fig. 3), was obtained from *PHY* cDNA by PCR amplification with the primers *GAF1FW* and *GAF1RV* (Tab. 2), while a 421 bp product, corresponding to the region 727 bp-1147 bp (Fig. 3), was generated using *GAFIFW* and *GAF2R* (Tab.2)

**Tab. 2 - Primer sequences**

| | |
|---|---|
| ***GAF1fw*** | 5'-ACTGAATCCAGCTATCTTGGCATGC-3' SEQ ID. No. 3 |
| ***GAF1rv*** | 5'-ACTTCTAGATCATTGTCGACAACAAT-3' SEQ ID. No. 4 |
| ***GAF2rv*** | 5'-GATTCTAGAGCTCATAGTGCACTGGC-3' SEQ ID. No. 5 |
| ***FcpBpfw-SacII*** | 5'-AGTCCGCGGAATCTCGCCTATTCATG-3' SEQ ID. No. 6 |
| ***phy1fw*** | 5'-GCGTACGCTGAACCATCATA -3' SEQ ID. No. 7 |
| ***phy1rv*** | 5'-GTTCCGCACGATTTCTACGA-3' SEQ ID. No. 8 |
| ***phy2fw*** | 5'-GAATTGTGCTGGGTGATCCT-3' SEQ ID. No. 9 |
| ***phy2rv*** | 5'-GTGAGAGCTTTGCGTGTTGA-3' SEQ ID. No. 10 |
| ***phy3fw*** | 5'-GACATCGGGCATGTGATAGT-3' SEQ ID. No. 11 |
| ***phy3rv*** | 5'-GCAATAGAGGTCCTCACAGCA-3' SEQ ID. No. 12 |
| ***RPSfw*** | 5'-GTGCAAGAGACCGGACATACC-3' SEQ ID. No. 13 |
| ***RPSrv*** | 5'-CGAAGTCAACCAGGAAACCAA-3' SEQ ID. No. 14 |

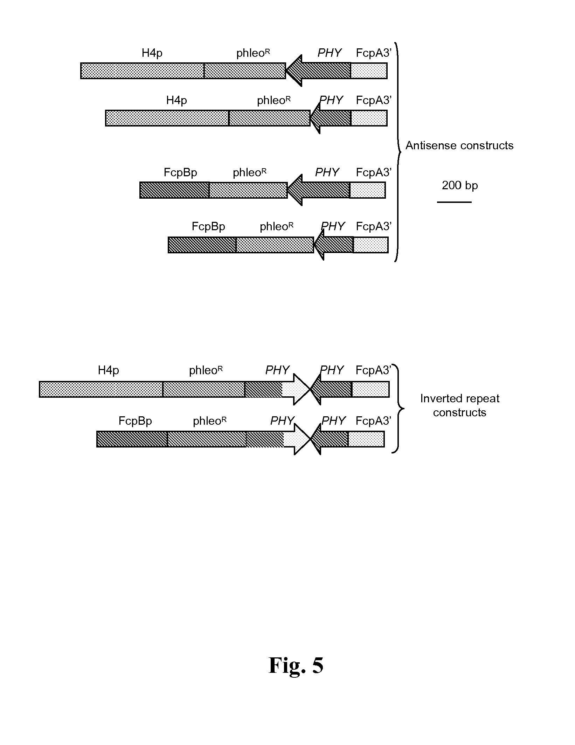

These two fragments had the first 254 bp in common. For the antisense construct, either the longer or the shorter *PHY* fragment was digested by *Eco*RI and *Xba*I and subsequently introduced in the antisense orientation, between the *Sh ble* gene and the FcpA terminator, into the *Eco*RI *- Xba*I linearized GUS antisense vector, replacing the GUS fragment (Fig. 3). For the inverted-repeat construct, both the longer and the shorter fragments were digested by *Eco*RI and *Xba*I and subsequently ligated to each other, by means of the *Xba*I site, in sense and antisense orientations, respectively, and were cloned into the *Eco*RI linearized inverted-repeat vector (Fig. 5).

### Genetic transformation of P. tricornutum

The antisense and inverted-repeat vectors were introduced into the CCMP632 strain of *P. tricornutum* Bohlin by microparticle bombardment, using a Biolistic PDS-1000/He Particle Delivery System (Bio-Rad, Hercules, CA, USA) as described in (Falciatore et al. 1999).

Putative silenced clones were first selected on 50% fresh seawater (SW) agar plates (1% agar) supplemented with 50 µg/ml phleomycin (InVitrogen, San Diego, CA, USA). After ca. three weeks, individual resistant colonies were restreaked on 50% SW agar plates supplemented with phleomycin and then inoculated into liquid f/2 medium to be further analyzed.

### Screening of transformed clones and identification of silenced strains

In order to confirm the presence of the *phy* silencing constructs, an initial PCR screening of phleomicyn resistant clones was performed directly on diatom colonies (Falciatore et al. 1999). In particular a forward primer complementary to the FcpBp promoter has been used (*FcpBpfw-SacII*) and, as reverse primer complementary to the GAF domain *GAF1fw* (Tab.2). Amplification products were checked by electrophoresis on agarose gels. Afterwards a western blot analysis was performed on PCR positive clones to check the real amount of Pt Phy protein in comparison to the wild type. Proteins were extracted from a pellet corresponding to ca. 10⁷ cells in the exponential phase of growth, suspended in 45 µl of extraction buffer (Tris-HCl 50 mM pH 6.8, SDS 2%), vortexed and incubated at RT for 30 min. The soluble phase was recovered by centrifuging at 10,000 x g for 30 min at 4°C. The protein extract was spectrophotometrically quantified using the BCA reagent (BCA Protein Assay kit; Thermo Scientific) in accordance to the manufacturer's instructions. 50 µg of total proteins were separated in a 10% sodium dodecyl sulphate-polyacrylamide gel electrophoresis (SDS_PAGE) and transferred to a nitrocellulose membrane (POTRAN BA85; Schleicher & Schuell BioScence GmbH, Whatman Group, Dassel, Germany). The membrane was saturated with PBS 1X, Tween 0.1%, lyophilized milk 5% (1h at RT), then first incubated overnight with rabbit anti-Phy antibody (dilution 1: 1,000) (DeRiso et al. 2009) and subsequently with goat (HRP)-conjugated anti-rabbit secondary antibody IgG (H+L) (dilution 1:10,000) (Promega), for 1h at RT. Phy was revealed by using an Amersham enhanced chemiluminescence kit (ECL kit; Amersham Biosciences, Piscataway, NJ, USA). The anti-Cpf1 (antibody was utilized as loading control).

### Microscopic observation of silenced clones

The most strongly silenced clones were inoculated into liquid f/2 medium supplemented with 50 µg/ml phleomycin and incubated at 18 °C under cool white fluorescent lights (either 100 or 25 µmol·m⁻²·s⁻¹), in a 12h:12h dark-light cycle.) Cell cultures were observed by optical and SEM electron microscopes (Electron Microscope Service SZN).

### Sedimentation features

Sedimentation rate was quantified spectrophotometrically, by following the optical density (OD) decrease over the time (up to 2 hours) of the wild-type and the mutants, as the O.D₆₇₂ nm is proportional to the cell in suspension. The sedimentation rate was calculated for each strain considering that 100% of the cells were in suspension at the time 0. The OD was measured every 15 minutes for 2 hours. The slope of the curve indicates the rate of sedimentation.

### RESULTS

### phy knock-down mutants

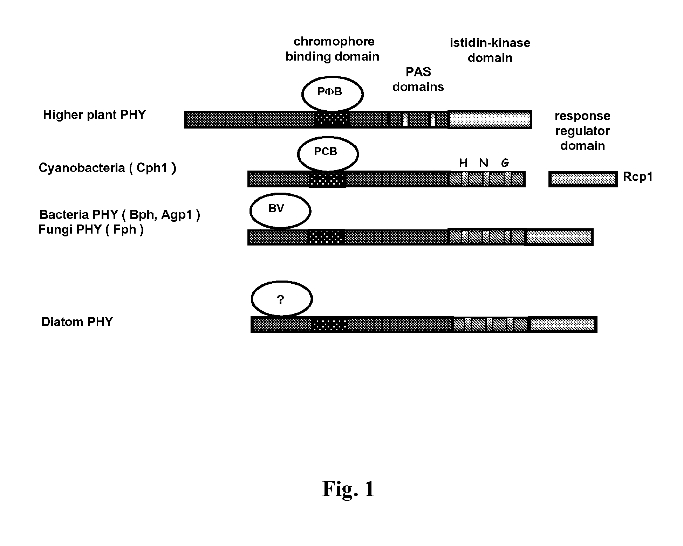

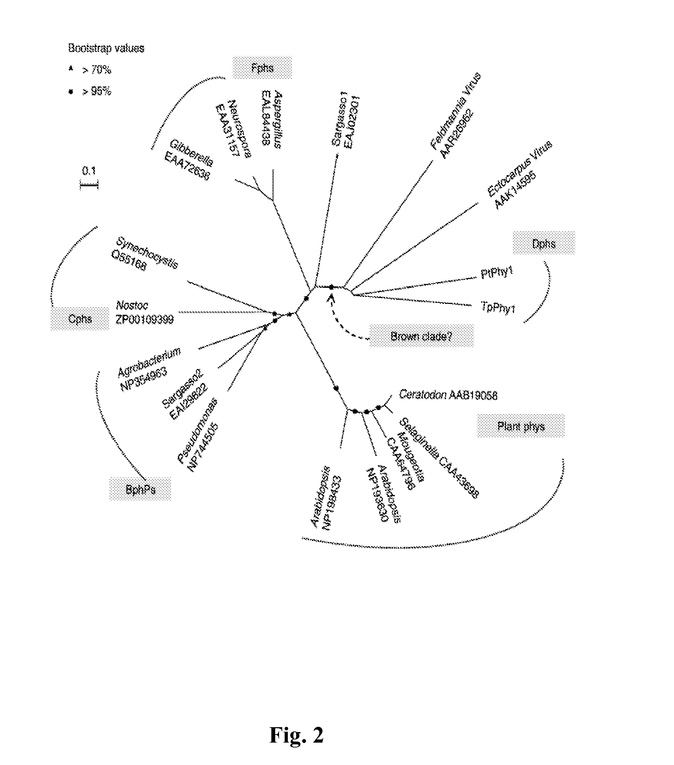

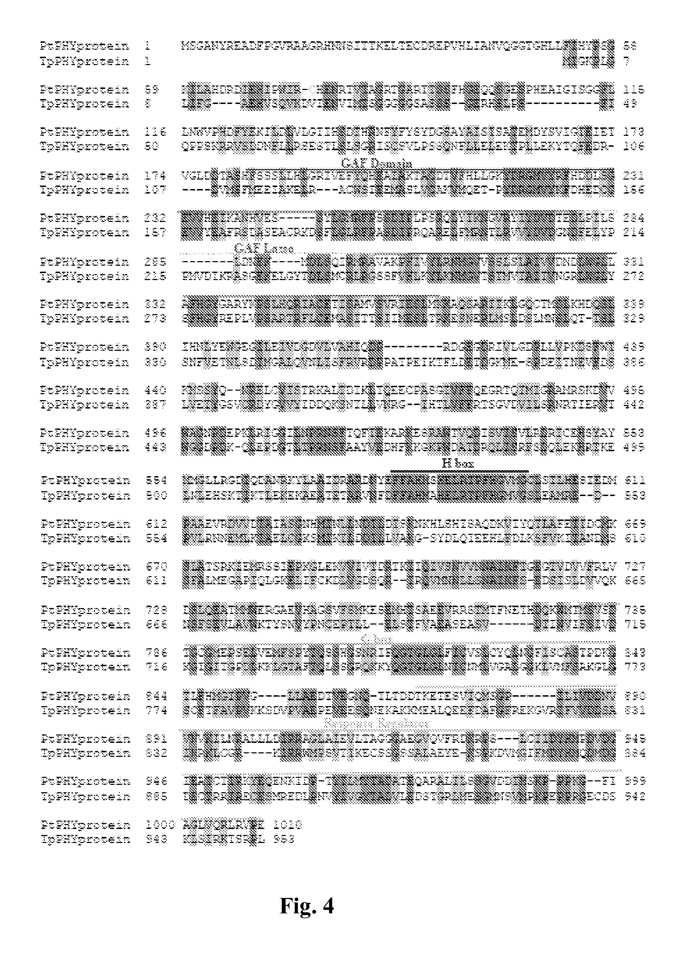

Phytochromes (Phys) belong to an extended family of photoreceptors widely distributed in photosynthetic and non-photosynthetic bacteria, fungi, algae and higher as well lower plants, living in very different environments (Montgomery and Lagarias 2002). Typically, red/far red responses are mediated by two photoconvertible phytochrome forms: Pr and Pfr, absorbing, respectively, red and far-red light (Chen et al. 2004). The overall similarity between phytochromes identified in different organisms appears in the GAF domain containing the putative chromophore binding site (Fig 1). All Phys utilize a linear tetrapyrrole as chromophore: plants incorporate phytochromobilin (PΦB), some cyanobacteria use phycocyanobilin (PCB), and bacteriophytochromes (BphP) contain the biliverdin precursor (BV). Recently, it has been shown that bacteriophytochromes isolated from some photosynthetic bacteria do not bind a chromophore. This loss of light sensing is replaced by a redox-sensing ability coupled to a kinase activity (Vuillet et al. 2007). Phytochrome-like proteins have been identified in the genomes of the marine diatom *Phaeodactylum tricornutum* (http://genome.jgi-psf.org/cgi-bin/dispTranscript?db=Phatr2&id=54330&useCoords=1) and *Thalassiosira pseudonana* (http://genome.jgi-psf.org/Thaps3/Thaps3.home.html). Interestingly, the analysis of the diatom protein sequence indicates a putative N-terminal chromophore binding domain, followed by histidine kinase and response regulator (RR) modules at the C-terminus (Montsant et al. 2007). This structure makes the diatom Phy more similar to bacteriophytochrome (BphP) than to plant Phy; moreover diatom Phy contains a Cys residue at the N-terminus of the protein, as in bacteriophytochrome, that might be involved in the covalent linkage of the putative chromophore allowing for a light-activated kinase similar to bacterial two-component sensors (Fig 2, 3 and 4) (Falciatore and Bowler, 2005). In order to clarify diatom phytochrome function, the authors have recently generated *phy* knock-down mutants in *P. tricornutum* by the RNA interference approach (De Riso et al., 2009). As reported in the methods, the authors have produced different constructs containing either antisense or inverted repeat fragments of the *PHY* gene (Fig. 5). In particular, a fragment from the GAF region of the *PHY* gene has been cloned between the 3' end of the selectable *Sh ble* gene, conferring resistance to phleomycin, and the terminator region of the diatom FcpA gene (Falciatore et al. 1999). This strategy allowed for a unique transcriptional product, with a consequent more efficient and simplified screening of silenced clones by their incubation on a selective medium.

Different *P. tricornutum* promoters were tested to successfully drive the transcription of silencing constructs: FcpBp and H4p, promoters from the Fucoxanthin Chlorophyll a/c-binding Protein B and Histone 4 genes, respectively. Their sequence is reported below.

### FcpBp sequence:

**H4p sequence**

The light-regulated FcpB promoter is routinely used to obtain strong expression of transgenes in diatoms, whereas H4p has been recently identified as a good candidate to drive constitutive expression (Siaut et al. 2007).

Concerning the inverted repeat construct, it was expected to encode for RNAs that fold into hairpin structures. In fact two *PHY* fragments (the 254 bp and the 421 bp fragment as indicated above) were introduced with an opposite orientation into the vector and were able to generate a loop of 167 bp after the annealing of their complementary regions of 254 bp in length (Fig. 5).

The different constructs were introduced into the CCMP632 strain of *P. tricornutum* by stable nuclear transformation (Falciatore et al. 1999). Putative silenced clones were initially selected on phleomycin. A higher number of clones were obtained by the transformation with constructs under FcpBp (77 clones), while only 30 clones were obtained by transforming with constructs driven by H4p (Tab 1).

**Tab. 1. Clones**

| | **Phleo^{R} clones** | **Western blot +** | **Stable clones** | **Phenotype** + |
|---|---|---|---|---|
| **F-ir** | 67 | 16 | 3 | 1 |
| **F-an** | 10 | 2 | - | - |
| **H-ir** | 12 | - | - | - |
| **H-an** | 18 | 3 | 2 | 2 |

| | | | | |
|---|---|---|---|---|
| **Phleo^{R} clones:** number of pheomycin resistant clones; **Western blot** +: clones with a significant decrease in Phy content (revealed by wester-blot); **Stable clones:** clones with an unchanged level of PHY silencing after nine months; **Phenotype** +: clones with a peculiar phenotype revealed by microscope observations. F-IR, vector containing the sense (254 bp)/antisense (421) fragments under the FcpBp; F-AN, vector containing the antisense (421 bp) fragment under the FcpBp; H-IR, vector containing the sense (254 bp)/antisense (421) fragments under the H4p; H-AN, vector containing the antisense (254 bp) fragment under the H4p. | | | | |

A preliminary PCR screening has been performed (as reported in M&M) to check for the presence of the silencing constructs. Afterwards, the content of Phy protein relative to that of the wild type was verified by western blot using a specific antibody in the clones positive in the PCR analysis.

Three clones whose transgene expression was driven by the H4 promoter (containing constructs with the antisense fragment of 254 bp) showed a significant reduction in Dph1 content by western blot. However, only in two clones, H-an6 and H-an4 (containing the 254 bp antisense) the protein reduction was stable after one year and independent of the light conditions (Tab 1).

In the case of the 421 bp antisense constructs driven by FcpBp, two silenced clones were identified but the protein reduction was transient for both of them (Tab. 1). The highest number of positive clones were identified with the inverted repeat construct under the FcpB promoter (16 clones), but in this case three clones (F-ir15, F-ir20 and F-ir26) showed stable silencing, revealing unchanged protein levels after one year (Tab. 1),

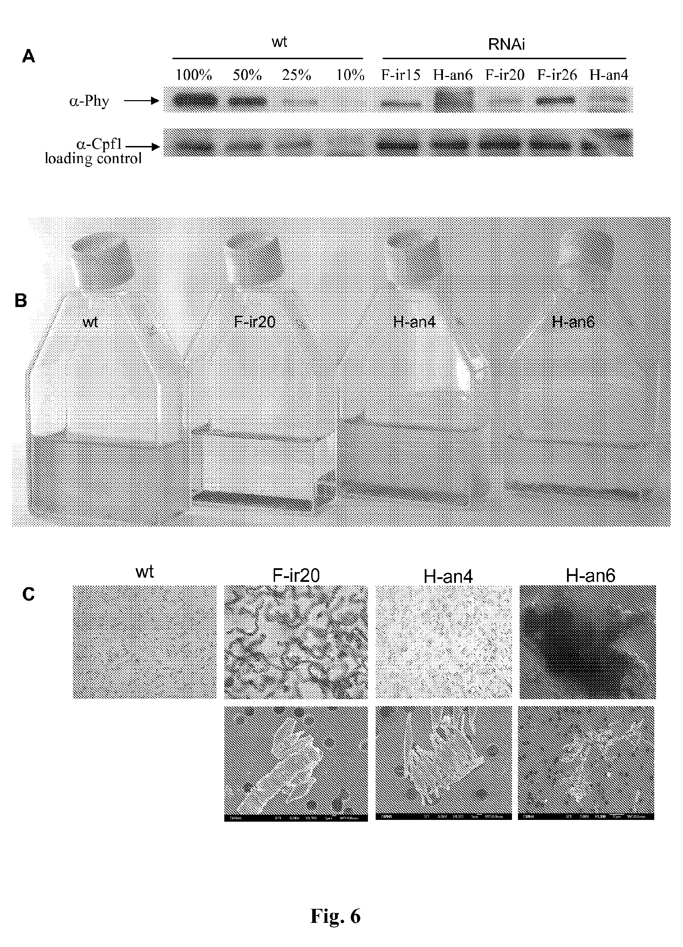

In conclusion, five clones showed a stable and significant reduction in Phy content in comparison to the wild type; in particular, two clones (F-ir15 and F-ir26), transformed with the inverted repeat construct under FcpBp, showed a protein decrease of ca. 50% in comparison to the wild type, two clones, F-ir20 and H-an4 (254 bp) revealed a reduction of ca. 75% and another one (H-an6), containing a 254 bp antisense construct driven by the H4p, showed a decrease of ca. 90% (Fig. 6A). The same results, also in terms of silencing extent, were found for all the five mutants by western blot after one year, demonstrating the stability of silencing.

Comparison of the relative *PHY* expression levels determined by qRT-PCR in the wild type and in the silenced clones revealed the absence of a significant correlation between the decrease in the protein content of transformed cells and a decline in the *PHY* mRNA content (data not shown), suggesting a possible inhibition at the translational level (De Riso et al. 2009).

### Characterization of aggregation phenotype of silenced clones

Interestingly, observations of the most silenced clones (F-ir20 and H-an6, H-an4) highlighted particular phenotypes in comparison to wild type *P. tricornutum* cells. Already by looking at the mutants grown in liquid medium, it was possible to observe sedimentation of the cells to the bottom of the flask (Fig. 6B). A more accurate analysis by microscopic observation revealed particular morphologies and aggregation phenotypes in the mutants.

In F-ir20 and H-an6, showing a Phy decrease of respectively 75% and 90%, the cell morphology shifted from the tapering shape typical of wild type cells to a characteristic ovoid shape (Fig. 6C). More interestingly, in all the three more silenced clones (F-ir20, H-an6 and H-an4) a particular and uncommon aggregation of cells was generated. The F-ir20 and the H-an4 clones showed the generation of chains, with the cells associated along their major axis. In the case of H-an6 the cells were strongly and chaotically aggregated even if in the less dense aggregates also the presence of short cell chains was apparent (Fig. 6). Preliminary analysis suggests that the level of aggregation correlates with cell division, and in fact the chains become longer with increasing division rates. In addition, a higher level of aggregation could be reached by growing the cells under optimal growth conditions. Moreover, investigation by electron microscopy revealed the strong adhesion between the valves of adjacent cells in the chains and the presence of uncharacterised polymeric materials (presumably polysaccharides) around and between cells of H-an6 (Fig. 6 C).

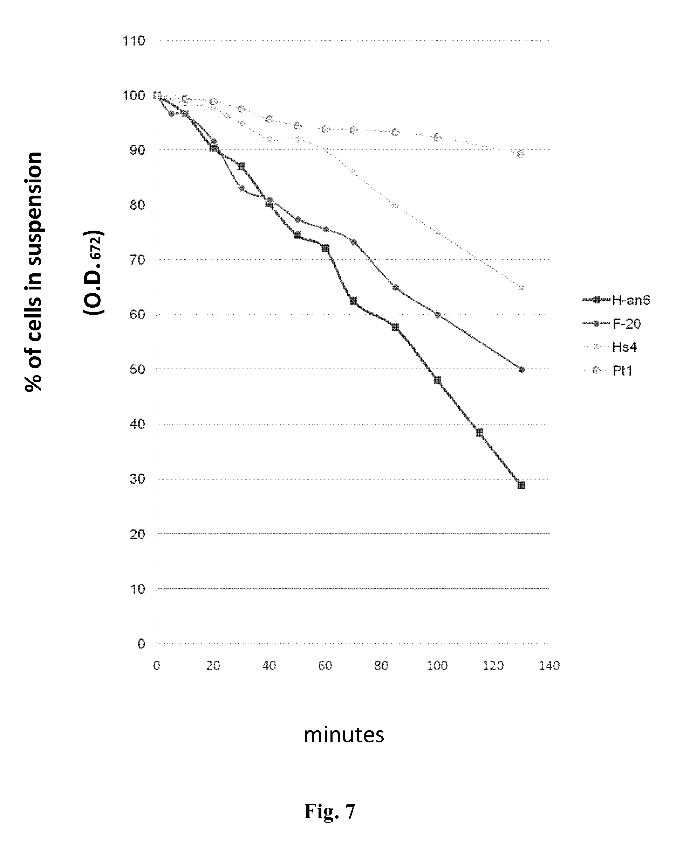

In order to better quantify the effect of the PHY silencing on the sedimentation, we followed the optical density variations over the time of the wild-type and the mutants, as the O.D₆₇₂ nm is proportional to the cell in suspension. As shown in Fig. 7, all the mutants showed a sedimentation rate faster than the wild types, and proportional to the silencing level. In particular, after two hour of measurement were still in suspensions: 95% of the wild-type cells, 65 % of the H-an4, 50% of F-ir20 and 30% of the H-an6.

### BIBLIOGRAPHIC REFERENCES

Armbrust EV, et al. (2004) The genome of the diatom Thalassiosira pseudonana: ecology, evolution, and metabolism. Science 306(5693):79-86
Bowler C, Allen AE, Badger JH, Grimwood J, Jabbari K, Kuo A, Maheswari U, (2008) The Phaeodactylum genome reveals the evolutionary history of diatom genomes. Nature 456(7219):239-44
Carrieri D, Bennette N, Ananyev GM, Posewitz M.C. (2008) Aquatic phototrophs: efficient alternatives to land-based crops for biofuels. Curr Opin Biotechnol 19(3):235-240
Chen M, Chory J, Fankhauser C (2004) Light signal transduction in higher plants. Annu Rev Genet 38: 87-117
Chisti Y (2007) Biodiesel from microalgae. Biotechnol Adv 25(3):294-306.
Chisti, Y (2008) Biodiesel from microalgae beats bioethanol. Trends Biotechnol 26:126-131
De Riso V, Raniello R, Maumus F, Bowler C and Falciatore A (2009) RNA interference in the marine diatom Phaeodactylum tricornutum. Nucleic Acids Res. 1-12 doi:10.1093/nar/gkp448
Dismukes GC, Carrieri D, Bennette N, Ananyey GM, Posewitz MC (2008) Aquatic phototrophs: efficient alternatives to land-based crops for biofuels. Curr Opin Biotechnol 19:235-240
Dunstan GA, Volkman JK, Barrett SM, Leroi JM, Jeffrey SW (1994) Essential polyunsaturated fatty acids from fourteen species of diatom (Bacillariophyceae). Phytochemistry 35:155-161.
Falciatore A and Bowler C (2005) The evolution and function of blue and red light photoreceptors. Curr Top Dev Biol 68:317-350
Falciatore A, Casotti R, Leblanc C, Abrescia C and Bowler C (1999) Transformation of Non selectable Reporter Genes in Marine Diatom. Marine Biotech 1:239-251
Falkowski PG, Barber RT and Smetacek V (1998) Biogeochemical controls and feedbacks on ocean primary production. Science 281:200-206
Guillard RRL (1975) Culture of phytoplankton for feeding marine invertebrates. In Culture of Marine Invertebrates Animals. Edit by Smith WL, Chanley MH, New York, USA.
Lebeau T and Robert JM (2003) Diatom cultivation and biotechnologically relevant products. Part II: current and putative products. Appl Microbiol Biotechnol. 60(6):624-32
Montgomery BL, and Lagarias JC (2002) Phytochrome ancestry: Sensors of bilins and light. Trends Plant Sci. 7:357-366.
Montsant A, Andrew EA, Coesel S, De Martino A, Falciatore A, Heijde M, Jabbari K, Maheswari U, Mangogna M, Rayko E et al (2007) Identification and comparative genomic analysis of signaling and regulatory mechanisms in the diatom Thalassiosira pseudonana. J of Phycol 43:585-604.
Sambrook J, Fritsch EF, Maniatis T (1989) Molecular cloning: A laboratory manual. Cold Spring Harbor Laboratory Press, Cold Spring Harbor, NY
Siaut M, Heijde M, Mangogna M, Montsant A, Coesel S, Allen AE, Manfredonia A (2007). Molecular toolbox for studying biology in Phaeodactylum tricornutum. Gene 406:23-35.
Vuillet L, Kojadinovic M, Zappa S, Jaubert M, Adriano JM, Fardoux J, Hannibal L (2007) Evolution of a bacteriophytochrome from light to redox sensor. EMBO J 26(14):3322-31
Zaslavskaia LA, Lippmeier JC, Shih C, Ehrhardt D, Grossman AR, Apt KE (2001) Trophic conversion of an obligate photoautotrophic organism through metabolic engeneering. Science 292:2073 -2075.

## Claims

1. A method to induce alga and/or diatom cell bio-flocculation comprising the step of inactivating the expression of at least one photoreceptor in said alga and/or diatom, wherein the photoreceptor is a phytocrome.

2. The method of claim 1 wherein the alga is selected from any of the following groups: the species Chromalveolata and Archaeplastida or the group of Bacillariophyceae.

3. The method of claim 1 wherein the diatom is selected from the species *Thalassiosira pseudonana* or *Phaeodactylum tricornutum.*

4. The method of any one of previous claims wherein the phytochrome is the Phy protein, essentially consisting of SEQ ID. No. 2.

5. The method of any one of previous claims wherein the inactivation of the expression of the photoreceptor is achieved by means of genetic engineering.

6. The method of claim 5 wherein the genetic engineering comprises the step of transforming the alga and/or the diatom cell with a nucleic acid construct able to inactivate the expression of the photoreceptor.

7. The method of claim 6 wherein the nucleic acid construct able to inactivate the expression of the photoreceptor comprises an alga and/or diatom cell active promoter operatively linked to a nucleic acid fragment of the photoreceptor cDNA.

8. The method of claim 7 wherein the nucleic acid construct is able to inactivate the expression of the photoreceptor Phy protein, essentially consisting of SEQ ID. No. 2, comprising a diatom active promoter region and operatively linked to a nucleic acid fragment of the *PHY* cDNA comprised between nt 727 and nt 980 of SEQ ID No. 1 or between nt 727 and nt 1147 of SEQ ID No. 1 introduced in the antisense orientation with respect to the nucleic acid fragment of the *PHY* cDNA.

9. The method of claim 8 wherein the promoter region is selected from the promoter region of the Fucoxanthin Chlorophyll a/c-binding Protein B gene (ID number 56299, SEQ ID. No. 15) or of the Histone 4 gene (ID number 34971, SEQ ID. No. 16).

10. Use of an alga and/or diatom cell transformed with a DNA construct as defined in claim 8 or 9 for the production of biodiesel and/or natural or recombinant molecules.

11. Use of the transformed alga and/or diatom cell transformed with a DNA construct as defined in claim 8 or 9 for the bioremediation of contaminated water or water enriched in phosphate and/or nitrogen.

12. Use of the alga and/or diatom cell transformed with a DNA construct as defined in claim 8 or 9 as feed for livestock or poultry.

13. Use of the transformed alga and/or diatom cell according to any of claims 10-12 , wherein the alga cell is selected from the species Chromalveolata and Archaeplastida or from the group of Bacillariophyceae.

14. Use of the transformed alga and/or diatom cell according to any of claim 10-12, wherein the diatom cell is selected from the species *Thalassiosira pseudonana* or *Phaeodactylum tricornutum.*

## Patentansprüche

1. Verfahren zum Induzieren der Bioflockulierung von Algen- und/oder Diatomeenzellen, das den Schritt des Inaktivierens der Expression mindestens eines Fotorezeptors in der Alge und/oder Diatomee umfasst, wobei der Fotorezeptor ein Phytochrom ist.

2. Verfahren nach Anspruch 1, wobei die Alge aus einer der folgenden Gruppen ausgewählt wird:
den Arten Chromalveolata und Archaeplastida oder der Gruppe der Bacillariophyceen.

3. Verfahren nach Anspruch 1, wobei die Diatomee ausgewählt ist aus den Arten *Thalassiosira pseudonana* oder *Phaeodactylum tricornutum.*

4. Verfahren nach einem der vorherigen Ansprüche, wobei das Phytochrom das Phy Protein ist, im Wesentlichen bestehend aus SEQ ID NO: 2.

5. Verfahren nach einem der vorherigen Ansprüche, wobei die Inaktivierung der Expression des Fotorezeptors durch Gentechnologie erreicht wird.

6. Verfahren nach Anspruch 5, wobei die Gentechnologie den Schritt des Transformierens der Algen- und/oder Diatomeenzelle mit einem Nukleinsäurekonstrukt umfasst, das in der Lage ist, die Expression des Fotorezeptors zu inaktivieren.

7. Verfahren nach Anspruch 6, wobei das Nukleinsäurekonstrukt, das in der Lage ist, die Expression des Fotorezeptors zu inaktivieren, einen in Algen- und/oder Diatomeenzellen aktiven Promotor umfasst, der operativ mit einem Nukleinsäurefragment der Fotorezeptor cDNA verknüpft ist.

8. Verfahren nach Anspruch 7, wobei das Nukleinsäurekonstrukt in der Lage ist, die Expression des Fotorezeptor Phy Proteins zu inaktivieren, das im Wesentlichen aus der SEQ ID NO: 2 besteht, umfassend eine in Diatomeen aktive Promotorregion und operativ damit verknüpft ein Nukleinsäurefragment der *PHY* cDNA umfasst zwischen Nt 727 und Nt 980 von SEQ ID NO: 1 oder zwischen Nt 727 und Nt 1147 von SEQ ID NO: 1, das in Bezug auf das Nukleinsäurefragment der PHY cDNA in Antisinn-Orientierung eingeführt wird.

9. Verfahren nach Anspruch 8, wobei die Promotorregion ausgewählt wird aus der Promotorregion des Gens des Fucoxanthin Chlorophyll a/c-bindenden Proteins B (ID Nummer 56299, SEQ ID NO: 15) oder des Histon 4 Gens (ID Nummer 34971, SEQ ID NO: 16).

10. Verwendung einer Algen- und/oder Diatomeenzelle, die mit einem DNA Konstrukt wie in Anspruch 8 oder 9 definiert transformiert wurde, zur Herstellung von Biodiesel und/oder natürlichen oder rekombinanten Molekülen.

11. Verwendung der Algen- und/oder Diatomeenzelle, die mit einem DNA Konstrukt wie in Anspruch 8 oder 9 definiert transformiert wurde, zur biologischen Aufbereitung von kontaminiertem Wasser oder Wasser, das mit Phosphat und/oder Stickstoff angereichert ist.

12. Verwendung der Algen- und/oder Diatomeenzelle, die mit einem DNA Konstrukt wie in Anspruch 8 oder 9 definiert transformiert wurde, als Futter für Vieh oder Geflügel.

13. Verwendung der transformierten Algen- und/oder Diatomeenzelle nach einem der Ansprüche 10-12, wobei die Algenzelle ausgewählt ist aus den Arten Chromalveolata und Archaeplastida oder aus der Gruppe der Bacillariophyceen.

14. Verwendung der transformierten Algen- und/oder Diatomeenzelle nach einem der Ansprüche 10-12, wobei die Diatomeenzelle ausgewählt ist aus den Arten *Thalassiosira pseudonana* oder *Phaeodactylum tricornutum.*

## Revendications

1. Procédé d'induction de bio-floculation cellulaire d'algue et/ou de diatomée comprenant l'étape d'inactivation de l'expression d'au moins un photorécepteur dans ladite algue et/ou ladite diatomée, dans lequel le photorécepteur est un phytochrome.

2. Procédé selon la revendication 1, dans lequel l'algue est sélectionnée dans l'un quelconque des groupes suivants :
les espèces Chromalveolata et Archaeplastida ou le groupe des Bacillariophyceae.

3. Procédé selon la revendication 1, dans lequel la diatomée est sélectionnée parmi les espèces *Thalassiosira pseudonana* ou *Phaeodactylum tricornutum.*

4. Procédé selon l'une quelconque des revendications précédentes, dans lequel le phytochrome est la protéine Phy, essentiellement constituée de SEQ ID NO : 2.

5. Procédé selon l'une quelconque des revendications précédentes, dans lequel l'inactivation de l'expression du photorécepteur est obtenue par génie génétique.

6. Procédé selon la revendication 5, dans lequel le génie génétique comprend l'étape de transformation de la cellule d'algue et/ou de diatomée avec une construction d'acide nucléique capable d'inactiver l'expression du photorécepteur.

7. Procédé selon la revendication 6, dans lequel la construction d'acide nucléique capable d'inactiver l'expression du photorécepteur comprend un promoteur actif dans les cellules d'algues et/ou de diatomées fonctionnellement lié à un fragment d'acide nucléique de l'ADNc du photorécepteur.

8. Procédé selon la revendication 7, dans lequel la construction d'acide nucléique est capable d'inactiver l'expression de la protéine Phy photoréceptrice, essentiellement constituée de SEQ ID NO : 2, comprenant une région promotrice active de diatomée et fonctionnellement liée à un fragment d'acide nucléique de l'ADNc de *PHY* compris entre nt 727 et nt 980 de SEQ ID NO : 1 ou entre nt 727 et nt 1147 de SEQ ID NO : 1 introduit dans l'orientation antisens relativement au fragment d'acide nucléique de l'ADNc de *PHY.*

9. Procédé selon la revendication 8, dans lequel la région promotrice est sélectionnée parmi la région promotrice du gène de la protéine B de liaison à la Fucoxanthine Chlorophile a/c (numéro d'ID 56299, SEQ ID NO : 15) ou du gène de l'histone 4 (numéro d'ID 34971, SEQ ID NO : 16).

10. Utilisation d'une cellule d'algue et/ou de diatomée transformée avec une construction d'ADN comme définie dans la revendication 8 ou la revendication 9, pour la production de biodiesel et/ou de molécules naturelles ou recombinantes.

11. Utilisation de la cellule d'algue et/ou de diatomée transformée, transformée avec une construction d'ADN comme définie dans la revendication 8 ou la revendication 9, pour la bioréhabilitation d'eau polluée ou d'eau enrichie en phosphate et/ou en azote.

12. Utilisation de la cellule d'algue et/ou de diatomée transformée avec une construction d'ADN comme définie dans la revendication 8 ou la revendication 9, en tant qu'aliment pour le bétail ou la volaille.

13. Utilisation de la cellule d'algue et/ou de diatomée transformée selon l'une quelconque des revendications 10 à 12, dans laquelle la cellule d'algue est sélectionnée parmi les espèces Chromalveolata et Archaeplastida ou dans le groupe des Bacillariophyceae.

14. Utilisation de la cellule d'algue et/ou de diatomée transformée selon l'une quelconque des revendications 10 à 12, dans laquelle la cellule de diatomée est sélectionnée parmi les espèces *Thalassiosira pseudonana* ou *Phaeodactylum tricornutum.*
